# EUROPEAN PATENT SPECIFICATION

(11) **EP 3 528 837 B1**
(45) Date of publication and mention of the grant of the patent: **10.06.2026**
(21) Application number: 17794266.1
(22) Date of filing: 20.10.2017
(51) Int. Cl.: A61K 38/47, C12N 9/36

(54) **NEW ANTIMICROBIAL AGENTS AGAINST ENTEROCOCCUS BACTERIA**
NEUE ANTIMIKROBIELLE WIRKSTOFFE GEGEN ENTEROKOKKENBAKTERIEN
NOUVEAUX AGENTS ANTIMICROBIENS CONTRE LES BACTÉRIES ENTÉROCOQUES

(30) Priority: 20.10.2016 EP 16194788
(43) Date of publication of application: 28.08.2019
(73) Proprietor: Lysando AG, 9497 Triesenberg (LI)
(72) Inventor: BIEBL, Manfred, 93083 Obertraubling (DE); SCHIRMEIER, Eva, 93053 Regensburg (DE); GRIESSL, Martin, 93155 Hohenschambach (DE)
(74) Representative: Rückerl, Florian
(86) International application number: PCT/EP2017/076861
(87) International publication number: WO 2018/073416

(56) References cited:
- WO-A1-2010/149795
- WO-A1-2014/052438
- WO-A1-91/19512
- US-A1- 2007 025 978
- YOONG PAULINE ET AL: "Identification of a broadly active phage lytic enzyme with lethal activity against antibiotic-resistant Enterococcus faecalis and Enterococcus faecium", JOURNAL OF BACTERIOLOGY, vol. 186, no. 14, 1 July 2004 (2004-07-01), , AMERICAN SOCIETY FOR MICROBIOLOGY, US, pages 4808 - 4812, XP009136845, ISSN: 0021-9193
- DATABASE UniProt [online] 16 August 2004 (2004-08-16), "SubName: Full=N-acetylmuramoyl-L-alanine amidase {ECO:0000313|EMBL:AAT01859.1};", retrieved from EBI accession no. UNIPROT:Q6E4B1 Database accession no. Q6E4B1
- DATABASE Protein [online] 14 June 2014 (2014-06-14), ANONYMOUS: "holin [Streptococcus suis]", XP002776751, retrieved from NCBI accession no. WP_029171101 Database accession no. WP_029171101
- QIUHUA DONG ET AL: "Construction of a chimeric lysin Ply187N-V12C with extended lytic activity against staphylococci and streptococci : Chimeric lysin Ply187N-V12C with extended lytic activity", MICROBIAL BIOTECHNOLOGY, vol. 8, no. 2, 15 September 2014 (2014-09-15), GB, pages 210 - 220, XP055433293, ISSN: 1751-7915, DOI: 10.1111/1751-7915.12166

## Description

The present invention relates to the field of antimicrobial agents active against *Enterococcus* bacteria. In particular, the present invention relates to a polypeptide comprising i) the amino acid sequence of SEQ ID NO: 101; ii) the amino acid sequence of SEQ ID NO: 102; iii) the amino acid sequence of SEQ ID NO: 103; or iv) the amino acid sequence of SEQ ID NO: 105. In addition, the present invention relates to nucleic acids encoding such polypeptides, vectors comprising such nucleic acids, and corresponding host cells. Finally, the present invention relates to applications of the inventive polypeptides, nucleic acids, vectors, and/or host cells, in particular in the pharmaceutical field.

Bacterial pathogens represent a significant threat for human health. Although various types of agents having bactericidal or bacteriostatic activity are known in the art (e.g. antibiotics), microbial resistance to these, in particular to antibiotics, is steadily increasing. One of the pathogens representing a health concern are some bacteria of the genus *Enterococcus.* They can cause life-threatening infections in humans, especially in the nosocomial (hospital) environment. Particularly relevant are in this context *Enterococcus faecalis* and *Enterococcus faecium* bacteria, which account for more than 90% of the infections. Enterococcus *faecalis* and Enterococcus *faecium* are Gram-positive bacteria that commensally colonize the lower intestinal tract, oral cavity, and vaginal tract of humans. In healthy individuals, *E. faecalis* and *E. faecium* colonization normally has no adverse effect on the host; however, the acquisition of virulence factors and high-level antibiotic resistance by enterococci may cause severe problems, in particular in immunocompromised patients. Common diseases caused by enterococcal infections include endocarditis, abdominal abscesses, bacteremia, and urinary tract infections. Since increasing resistance diminishes the utility of conventional antibiotics, there is a constant demand for new antimicrobial agents to control the number of *Enterococcus* bacteria, e.g. in in the nosocomial (hospital) environment.

The present invention makes use of enzymes degrading the bacterial cell wall, such as an endolysin. Endolysins are peptidoglycan hydrolases typically encoded by bacteriophages (or bacterial viruses). They are synthesized during late gene expression in the lytic cycle of phage multiplication and mediate the release of progeny virions from infected cells through degradation of the bacterial peptidoglycan. They are either *N*-acetyl-β-D-muramidases (lysozymes), lytic transglycosylases, *N*-acetyl-β-D-glucosaminidases, *N*-acetylmuramoyl-L-alanine amidases or endopeptidases. Antimicrobial application of endolysins was already suggested in 1991 by Gasson (GB2243611). Although the killing capacity of endolysins has been known for a long time, the use of these enzymes as antibacterials was ignored due to the success and dominance of antibiotics. Only after the appearance of multiple antibiotic resistant bacteria this simple concept of combating human pathogens with endolysins received interest. A compelling need to develop totally new classes of antibacterial agents emerged and endolysins used as 'enzybiotics' - a hybrid term of 'enzymes' and 'antibiotics' - perfectly met this need. In 2001, Fischetti and coworkers demonstrated for the first time the therapeutic potential of bacteriophage Cl endolysin towards group A streptococci (Nelson et al., 2001, Proc. Natl. Acad. Sci. U. S. A. 98:4107-4112). Since then many publications have established endolysins as an attractive and complementary alternative to control bacterial infections of Gram-positive bacteria. Subsequently different endolysins against other Gram-positive pathogens such as *Streptococcus pneumoniae* (Loeffler et al., 2001, Science 294:2170-2172), *Bacillus anthracis* (Schuch et al., 2002; Nature 418:884-889), *S*. *agalactiae* (Cheng et al., 2005; Antimicrob Agents Chemother. 2005 Jan;49(1):111-117) and *Staphylococcus aureus* (Rashel et al, 2007; J Infect Dis. 2007 Oct 15;196(8):1237-1247) have proven their efficacy as enzybiotics.

In the art combinations of endolysins with further amino acid sequence stretches have been described to create new antimicrobial agents. WO 2010/149795 discloses fusions of peptides with derivatives of endolysin, which show activity towards various bacteria. However, such fusions have not been specifically described for *Enterococcus* bacteria.

Yoong et al (J Bacteriol. 2004 Jul;186(14):4808-4812) report the identification of PlyV12, a bacteriophage lytic enzyme that was shown to kill both *E. faecalis* and *E. faecium.*

US 2007/025978 A1 relates to bacteriophage endolysins useful for the reduction of *E. faecalis* and *E. faecium* (including vancomycin resistant strains).

WO 2014/052438 discloses methods for making and using antimicrobial peptides.

NCBI accession number WP_029171101 discloses the protein sequence of a holin protein identified in *S*. *suis.*

WO 91/19512 A1 discloses antimicrobial peptides which are related to sequences of Carthepsin G and which act against both gram-negative and gram-positive bacterial strains of *E. coli, N. gonorrhoeae, P. vulgaris, S. aureus, L. monocytogenes* and *P. gingivalis.*

Thus, there is still a constant need for new antibacterial agents active against Gram-positive bacteria. In particular, there is a need for antibacterial agents active against bacteria of the

Genus *Enterococcus.* Preferably, said agents are active against a diverse set of *Enterococcus* strains, exhibit an increased activity and/or are, e.g., sufficiently pH tolerant to allow broad utility in medical technology and pharmaceutical applications.

This object is solved by the subject matter defined in the claims and set forth below.

The term "polypeptide" as used herein refers in particular to a polymer of amino acids linked by peptide bonds in a specific sequence. The amino acid residues of a polypeptide may be modified by e.g. covalent attachments of various groups such as carbohydrates and phosphate. Other substances may be more loosely associated with the polypeptide, such as heme or lipid, giving rise to conjugated polypeptides which are also comprised by the term "polypeptide" as used herein. The term as used herein is intended to encompass also proteins. Thus, the term "polypeptide" also encompasses for example complexes of two or more amino acid polymer chains. The term "polypeptide" does encompass embodiments of polypeptides which exhibit optionally modifications typically used in the art, e.g. biotinylation, acetylation, pegylation, chemical changes of the amino-, SH- or carboxyl-groups (e.g. protecting groups) etc.. As will become apparent from the description below, the polypeptide according to the present invention may also be a non-naturally occurring polypeptide. For example, the polypeptide of the present invention may be a fusion protein, in which at least two amino acid sequences are combined, which do not occur in this combination in nature. The term "polypeptide", as used herein, is not limited to a specific length of the amino acid polymer chain, but typically the polypeptide will exhibit a length of more than about 50 amino acids, more than about 100 amino acids or even more than about 150 amino acids. Usually, but not necessarily, a typical polypeptide of the present invention will not exceed about 750 amino acids in length.

The term "endolysin" as used herein refers to a bacteriophage-derived enzyme which is suitable to hydrolyse bacterial cell walls. Endolysins comprise at least one "enzymatically active domain" (EAD) having at least one of the following activities: endopeptidase, chitinase, T4 like muraminidase, lambda like muraminidase, N-acetyl-muramoyl-L-alanine-amidase (amidase), muramoyl-L-alanine-amidase, muramidase , lytic transglycosylase (C), lytic transglycosylase (M), N-acetyl-muramidase (lysozyme), N-acetyl-glucosaminidase or transglycosylases. In addition, the endolysins may contain also regions which are enzymatically inactive, and bind to the cell wall of the host bacteria, the so-called CBDs (cell wall binding domains). The term "endolysin" also encompasses enzymes which comprise modifications and/or alterations vis-a-vis naturally occurring endolysins. Such alterations and/or modifications may comprise mutations such as deletions, insertions and additions, substitutions or combinations thereof and/or chemical changes of the amino acid residues. Particularly preferred chemical changes are biotinylation, acetylation, pegylation, chemical changes of the amino-, SH- or carboxyl- groups. Said endolysins exhibit on a general level the lytic activity of the respective wild-type endolysin. However, said activity can be the same, higher or lower as the activity of the respective wild-type endolysin. Said activity can be for example at least about 50, 60, 70, 80, 90, 100, 110, 120, 130, 140, 150, 160, 170, 180, 190 or at least about 200 % of the activity of the respective wild-type endolysin or even more. The activity can be measured by assays well known in the art by a person skilled in the art as e.g. antibacterial assays which are e.g. described in Briers et al. (J. Biochem. Biophys Methods; 2007; 70: 531-533) or Donovan et al. (J. FEMS Microbiol Lett. 2006 Dec;265(1) and similar publications.
respective parent molecule. Also preferred derivatives are those resulting from conservative amino acid substitutions within the parent sequence, for example a given SEQ ID NO, again retaining the activity of the parent molecule on a general level.

As used herein, the term "% sequence identity", has to be understood as follows: Two sequences to be compared are aligned to give a maximum correlation between the sequences. This may include inserting "gaps" in either one or both sequences, to enhance the degree of alignment. A % identity may then be determined over the whole length of each of the sequences being compared (so-called global alignment), that is particularly suitable for sequences of the same or similar length, or over shorter, defined lengths (so-called local alignment), that is more suitable for sequences of unequal length. In the above context, an amino acid sequence having a "sequence identity" of at least, for example, 95% to a query amino acid sequence, is intended to mean that the sequence of the subject amino acid sequence is identical to the query sequence except that the subject amino acid sequence may include up to five amino acid alterations per each 100 amino acids of the query amino acid sequence. In other words, to obtain an amino acid sequence having a sequence of at least 95% identity to a query amino acid sequence, up to 5% (5 of 100) of the amino acid residues in the subject sequence may be inserted or substituted with another amino acid or deleted. Methods for comparing the identity and homology of two or more sequences are well known in the art. The percentage to which two sequences are identical can for example be determined by using a mathematical algorithm. A preferred, but not limiting, example of a mathematical algorithm which can be used is the algorithm of Karlin et al. (1993), PNAS USA, 90:5873-5877. Such an algorithm is integrated in the BLAST family of programs, e.g. BLAST or NBLAST program (see also Altschul et al., 1990, J. Mol. Biol. 215, 403-410 or Altschul et al. (1997), Nucleic Acids Res, 25:3389-3402), accessible through the home page of the NCBI at world wide web site ncbi.nlm.nih.gov) and FASTA (Pearson (1 990), Methods Enzymol. 83, 63-98; Pearson and Lipman (1988), Proc. Natl. Acad. Sci. U. S. A 85, 2444-2448.). Sequences which are identical to other sequences to a certain extent can be identified by these programmes. Furthermore, programs available in the Wisconsin Sequence Analysis Package, version 9.1 (Devereux et al, 1984, Nucleic Acids Res., 387-395), for example the programs BESTFIT and GAP, may be used to determine the % identity between two polypeptide sequences. BESTFIT uses the "local homology" algorithm of (Smith and Waterman (1981), J. Mol. Biol. 147, 195-197.) and finds the best single region of similarity between two sequences. If herein reference is made to an amino acid sequence sharing a particular extent of sequence identity to a reference sequence, then said difference in sequence is preferably due to conservative amino acid substitutions. Preferably, such sequence retains the activity of the reference sequence, e.g. albeit maybe at a slower rate. In addition, if reference is made herein to a sequence sharing "at least" at certain percentage of sequence identity, then 100% sequence identity are preferably not encompassed.

"Conservative amino acid substitutions", as used herein, may occur within a group of amino acids which have sufficiently similar physicochemical properties, so that a substitution between members of the group will preserve the biological activity of the molecule (see e.g. Grantham, R. (1974), Science 185, 862-864). Particularly, conservative amino acid substitutions are preferably substitutions in which the amino acids originate from the same class of amino acids (e.g. basic amino acids, acidic amino acids, polar amino acids, amino acids with aliphatic side chains, amino acids with positively or negatively charged side chains, amino acids with aromatic groups in the side chains, amino acids the side chains of which can enter into hydrogen bridges, e.g. side chains which have a hydroxyl function, etc.). Conservative substitutions are in the present case for example substituting a basic amino acid residue (Lys, Arg, His) for another basic amino acid residue (Lys, Arg, His), substituting an aliphatic amino acid residue (Gly, Ala, Val, Leu, lie) for another aliphatic amino acid residue, substituting an aromatic amino acid residue (Phe, Tyr, Trp) for another aromatic amino acid residue, substituting threonine by serine or leucine by isoleucine. Further conservative amino acid exchanges will be known to the person skilled in the art.

The term "deletion" as used herein refers preferably to the absence of 1, 2, 3, 4, 5 (or even more than 5) continuous amino acid residues in the derivative sequence in comparison to the respective reference sequence, either intrasequentially or at the N- or C-terminus. A derivative of the present invention may exhibit one, two or more of such deletions.

The term "insertion" as used herein refers preferably to the additional intrasequential presence of 1, 2, 3, 4, 5 (or even more than 5) continuous amino acid residues in the derivative sequence in comparison to the respective reference sequence. A derivative of the present invention may exhibit one, two or more of such insertions.

The term "addition" as used herein refers preferably to the additional presence of 1, 2, 3, 4, 5 (or even more than 5) continuous amino acid residues at the N- and/or C-terminus of the derivative sequence in comparison to the respective reference sequence.

The term "substitution" as used herein refers to the presence of an amino acid residue at a certain position of the derivative sequence which is different from the amino acid residue which is present or absent at the corresponding position in the reference sequence. A derivative of the present invention may exhibit 1, 2, 3, 4, 5, 6, 7, 8, 9, 10, 11, 12, 13, 14, 15, 16, 17, 18, 19, 20, or more of such substitutions. As mentioned above, preferably such substitutions are conservative substitutions.

The term "second amino acid sequence", as used herein refers to an amino acid subsequence within the amino acid sequence of the polypeptide of the invention. Said sequence may be the sequence of a cationic peptide, a polycationic peptide, an amphipathic peptide, a hydrophobic peptide, a sushi peptide and/or an antimicrobial peptide. The term does not refer to conventional tags like His-tags, such as His5-tags, His6-tags, His7-tags, His8-tags, His9-tags, His10-tags, His11-tags, His12-tags, His16-tags and His20-tags, Strep-tags, Avi-tags, Myc-tags, Gst-tags, JS-tags, cystein-tags, FLAG-tags or other tags known in the art, thioredoxin or maltose binding proteins (MBP). Preferably, the second amino acid sequence has a length of at least about 6 to at most about 50, preferably at most about 39 amino acid residues. Preferably, the second amino acid sequence is heterologous to the first amino acid sequence, e.g. the two amino acid sequences do not occur together in a single polypeptide in nature. Moreover, the second amino acid sequence itself does not provide any of the following enzymatic activities: endopeptidase, chitinase, T4 like muraminidase, lambda like muraminidase, N-acetyl-muramoyl-L-alanine-amidase (amidase), muramoyl-L-alanine-amidase, muramidase , lytic transglycosylase (C), lytic transglycosylase (M), N-acetyl-muramidase (lysozyme), N-acetyl-glucosaminidase or transglycosylase. Typically, the second amino acid stretch will not provide any enzymatic activity at all.

The terms "first amino acid sequence" and "second amino acid sequence", as used herein, do not imply an inherent order of the sequences within the inventive polypeptide, i.e. the second amino acid sequence may be N-terminal of the first amino acid sequence or C-terminal of the first amino acid sequence. There may also be further, e.g. intervening, sequence elements.

As used herein, the term "cationic peptide" refers preferably to a peptide having positively charged amino acid residues. Preferably a cationic peptide has a pKa-value of 9.0 or greater. Typically, at least four of the amino acid residues of the cationic peptide can be positively charged, for example, lysine or arginine. "Positively charged" refers to the side chains of the amino acid residues which have a net positive charge at about physiological conditions. The term "cationic peptide" as used herein refers also to polycationic peptides, but also includes cationic peptides which comprise for example less than 20%, preferably less than 10% positively charged amino acid residues.

The term "polycationic peptide" as used herein refers preferably to a peptide composed of mostly positively charged amino acid residues, in particular lysine and/or arginine residues. A peptide is composed of mostly positively charged amino acid residues if at least about 20, 30, 40, 50, 60, 70, 75, 80, 85, 90, 95 or about 100 % of the amino acid residues are positively charged amino acid residues, in particular lysine and/or arginine residues. The amino acid residues being not positively charged amino acid residues can be neutrally charged amino acid residues and/or negatively charged amino acid residues and/or hydrophobic amino acid residues. Preferably the amino acid residues being not positively charged amino acid residues are neutrally charged amino acid residues, in particular serine and/or glycine.

The term, "antimicrobial peptide" (AMP) as used herein refers preferably to any naturally occurring peptide that has microbicidal and/or microbistatic activity on, for example, bacteria, viruses, fungi, yeasts, mycoplasma and protozoa. Thus, the term "antimicrobial peptide" as used herein refers in particular to any peptide having anti-bacterial, anti-fungal, anti-mycotic, anti-parasitic, anti-protozoal, anti-viral, anti-infectious, anti-infective and/or germicidal, algicidal, amoebicidal, microbicidal, bactericidal, fungicidal, parasiticidal, protozoacidal, protozoicidal properties. Preferred are anti-bacterial peptides. The antimicrobial peptide may be a member of the RNase A super family, a defensin, cathelicidin, granulysin, histatin, psoriasin, dermicidine or hepcidin. The antimicrobial peptide may be naturally occurring in insects, fish, plants, arachnids, vertebrates or mammals. Preferably the antimicrobial peptide may be naturally occurring in radish, silk moth, wolf spider, frog, preferably in Xenopus laevis, Rana frogs, more preferably in Rana catesbeiana, toad, preferably Asian toad Bufo bufo gargarizans, fly, preferably in Drosophila, more preferably in Drosophila melanogaster, in Aedes aegypti, in honey bee, bumblebee, preferably in Bombus pascuorum, flesh fly, preferably in Sarcophaga peregrine, scorpion, horseshoe crab, catfish, preferably in Parasilurus asotus, cow, pig, sheep, porcine, bovine, monkey and human. As used herein, an "antimicrobial peptide" (AMP) may in particular be a peptide which is not a cationic peptide, polycationic peptide, amphipathic peptide, sushi peptide, defensins, and hydrophobic peptide, but nevertheless exhibits antimicrobial activity.

The term "sushi peptide" as used herein refers to complement control proteins (CCP) having short consensus repeats. The sushi module of sushi peptides functions as a protein-protein interaction domain in many different proteins. Peptides containing a Sushi domain have been shown to have antimicrobial activities. Preferably, sushi peptides are naturally occurring peptides.

The term "defensin" as used herein refers to a peptide present within animals, preferably mammals, more preferably humans, wherein the defensin plays a role in the innate host defense system as the destruction of foreign substances such as infectious bacteria and/or infectious viruses and/or fungi. A defensin is a non-antibody microbicidal and/or tumoricidal protein, peptide or polypeptide. Examples for "defensins" are "mammalian defensins," alpha-defensins, beta-defensins, indolicidin and magainins. The term "defensins" as used herein refers both to an isolated form from animal cells or to a synthetically produced form, and refers also to variants which substantially retain the cytotoxic activities of their parent proteins, but whose sequences have been altered by insertion or deletion of one or more amino acid residues.

The term "amphipathic peptide" as used herein refers to peptides having both hydrophilic and hydrophobic functional groups. Preferably, the term "amphipathic peptide" as used herein refers to a peptide having a defined arrangement of hydrophilic and hydrophobic groups e.g. amphipathic peptides may be e.g. alpha helical, having predominantly non polar side chains along one side of the helix and polar residues along the rest of its surface.

The term "hydrophobic group" as used herein refers preferably to chemical groups such as amino acid side chains which are substantially water insoluble, but soluble in an oil phase, with the solubility in the oil phase being higher than that in water or in an aqueous phase. In

As used herein, the term "tag" refers to an amino acid sequence, which is typically in the art fused to or included in another amino acid sequence for a) improving expression of the overall amino acid sequence or polypeptide, b) facilitating purification of the overall amino acid sequence or polypeptide, c) facilitating immobilisation of the overall amino acid sequence or polypeptide, and/or d) facilitating detection of the overall amino acid sequence or polypeptide. Examples for tags are His tags, such as His5-tags, His6-tags, His7-tags, His8-tags, His9-tags, His10-tags, His11-tags, His12-tags, His16-tags and His20-tags, Strep-tags, Avi-tags, Myc-tags, GST-tags, JS-tags, cystein-tags, FLAG-tags, HA-tags, thioredoxin or maltose binding proteins (MBP), CAT, GFP, YFP, etc. The person skilled in the art will know a vast number of tags suitable for different technical applications. The tag may for example make such tagged polypeptide suitable for e.g. antibody binding in different ELISA assay formats or other technical applications.

As used herein, "about physiological pH" is intended to refer to a pH above 6.5, in particular a pH range of about 6.75 to about 8.5, more preferably 7 to 7.75, even more preferably 7.2 to 7.6 and even more preferably 7.3 to 7.5. Most preferably, physiological pH is a pH of about 7.4.

The term "comprising" as used herein shall not be construed as being limited to the meaning "consisting of" (i.e. excluding the presence of additional other matter). Rather, "comprising" implies that optionally additional matter may be present. The term "comprising" encompasses as particularly envisioned embodiments falling within its scope "consisting of" (i.e. excluding the presence of additional other matter) and "comprising but not consisting of" (i.e. requiring the presence of additional other matter), with the former being more preferred.

The inventors of the present invention have surprisingly found that the endolysin according to SEQ ID NO:1, and its derivatives, such as the "enzymatically active domain" (EAD; SEQ ID NO:2) or the cell wall binding domain (CBD, SEQ ID NO:3) thereof, are in combination with the antimicrobial peptide according to SEQ ID NO: 100 extremely useful components when designing antimicrobial agents against bacteria of the Genus *Enterococcus.* Such compounds show increased utility and activity, for example against *Enterococcus faecalis* bacteria, and/or are more pH tolerant than the wildtype endolysin (i.e. SEQ ID NO: 1). Particularly preferred polypeptides of the present invention will be in particular at about physiological pH (e.g. about pH 7.4) more active than the wildtype endolysin (i.e. SEQ ID NO:1) and will preferably exhibit essentially the same or even increased activity at about physiological pH as compared to more acidic pH (e.g. pH 5.25 or 6).

Therefore, the present invention relates in a first aspect to a polypeptide comprising:
i) the amino acid sequence according to SEQ ID NO: 101;
ii) the amino acid sequence according to SEQ ID NO:102;
iii) the amino acid sequence according to SEQ ID NO: 103; or
iv) the amino acid sequence according to SEQ ID NO: 105.
iii) an amino acid sequence according to SEQ ID NO: 5.

In embodiments in which the inventive polypeptide comprises the CBD (e.g. comprises SEQ ID NO:3 or derivatives thereof), it is preferred if the polypeptide comprises additionally the amino acid sequence of an enzyme capable of degrading the cell wall of bacteria, in particular of Gram positive bacteria. Said enzyme may be for example a vertebrate lysozyme (e.g. human or hen egg white lysozyme), but is preferably an endolysin, autolysin or bacteriocin (e.g. lysostaphin). Already Dong et al. (Microb Biotechnol. 2015 Mar;8(2):210-20) exemplified, that the cell wall binding domain (CBD) of SEQ ID NO:1 can be fused to many other lytic enzyme sequences, such as the catalytic domain (1-157aa) of lysin Ply187. The enzyme may also be the enzymatically active subunit of a holin of *Streptococcus suis.* The catalytic domain (1-157aa) of lysin Ply187 or the region covering amino acids 2-242 of NCBI Reference Sequence WP_029171101.1 and variants thereof, e.g. SEQ ID NO: 104, are possible embodiments of the present invention to be used in combination with SEQ ID NO:3 or derivatives thereof. In one embodiment, a polypeptide according to the present invention may for example comprise an amino acid sequence according to SEQ ID NO: 104 or a derivative of SEQ ID NO: 104 exhibiting at least 80%, at least 85%, at least 87,5%, at least 90%, at least 91%, at least 92%, at least 93%, at least 94%, at least 95%, at least 96%, at least 97%, at least 98%, at least 99%, or more than 99% sequence identity with SEQ ID NO:104. However, most preferably the inventive polypeptide comprises SEQ ID NO:2, as well as SEQ ID NO:3, or respective derivatives thereof.

Derivatives of SEQ ID NO:2, SEQ ID NO:3, or SEQ ID NO: 5, respectively, exhibit preferably at least 85%, at least 87,5%, at least 90%, at least 91%, at least 92%, at least 93%, at least 94%, at least 95%, at least 96%, at least 97%, at least 98%, at least 99%, or more than 99% sequence identity with SEQ ID NO:2, SEQ ID NO:3, or SEQ ID NO: 5, respectively.

For all embodiments discussed herein it is particularly preferred, that the inventive polypeptide comprises a derivative of SEQ ID NO:1. Particularly useful derivatives of SEQ ID NO:1 are SEQ ID NO: 5 (lacking the N-terminal methionine of SEQ ID NO:1) and derivatives thereof, as well as polypeptides with truncated sequences of SEQ ID NO:1 comprising SEQ ID NO:2 or SEQ ID NO:3, or respective derivatives thereof. Common to all these embodiments is that the inventive polypeptide does not comprise the amino acid sequence of SEQ ID NO:1.

The second amino acid sequence of the inventive polypeptide is selected from the group consisting of an antimicrobial peptide, amphipathic peptide, cationic peptide, polycationic peptide, hydrophobic peptide, sushi peptide or defensin.

Examples for cationic/ polycationic amino acid sequences are listed in the following table.

**Table 1:**

| **Amino acid sequence** | **Length** | **SEQ ID NO:** |
|---|---|---|
| KRKKRK | 6 | 6 |
| KRXKR | 5 | 7 |
| KRSKR | 5 | 8 |
| KRGSG | 5 | 9 |
| KRKKRKKRK | 9 | 10 |
| RRRRRRRRR | 9 | 11 |
| KKKKKKKK | 8 | 12 |
| KRKKRKKRKK | 10 | 13 |
| KRKKRKKRKKRK | 12 | 14 |
| KRKKRKKRKKRKKR | 14 | 15 |
| KKKKKKKKKKKKKKKK | 16 | 16 |
| KRKKRKKRKKRKKRKKRK | 18 | 17 |
| KRKKRKKRKKRKKRKKRKK | 19 | 18 |
| RRRRRRRRRRRRRRRRRRR | 19 | 19 |
| KKKKKKKKKKKKKKKKKKK | 19 | 20 |
| KRKKRKKRKRSKRKKRKKRK | 20 | 21 |
| KRKKRKKRKRSKRKKRKKRKK | 21 | 22 |
| KRKKRKKRKKRKKRKKRKKRK | 21 | 23 |
| KRKKRKKRKRGSGKRKKRKKRK | 22 | 24 |
| KRKKRKKRKRGSGSGKRKKRKKRK | 24 | 25 |
| KRKKRKKRKKRKKRKKRKKRKKRKK | 25 | 26 |
| KRKKRKKRKRSKRKKRKKRKRSKRKKRKKRK | 31 | 27 |
| KRKKRKKRKRGSGSGKRKKRKKRKGSGSGKRKKRKKRK | 38 | 28 |
| KRKKRKKRKKRKKRKKRKKRKKRKKRKKRKKRKKRKKRK | 39 | 29 |
| KRKKRKKRKRSKRKKRKKRKRSKRKKRKKRKRSKRKKRKKRK | 42 | 30 |

Examples for antimicrobial amino acid sequences which may be used in carrying out the present invention are listed in the following table.

**Table 2:**

| **Peptide** | **Sequence** | **SEQ ID NO** |
|---|---|---|
| LL-37 | LLGDFFRKSKEKIGKEFKRIVQRIKDFLRNLVPR TES | 31 |
| SMAP-29 | RGLRRLGRKIAHGVKKYGPTVLRIIRIAG | 32 |
| Indolicidin | ILPWKWPWWPWRR | 33 |
| Protegrin | RGGRLCYCRRRFCVCVGR | 34 |
| Cecropin P1 | SWLSKTAKKLENSAKKRISEGIAIAIQGGPR | 35 |
| Magainin | GIGKFLHSAKKFGKAFVGEIMNS | 36 |
| Pleurocidin | GWGSFFKKAAHVGKHVGKAALTHYL | 37 |
| Cecropin A (A. aegypti) | GGLKKLGKKLEGAGKRVFNAAEKALPVVAGAKAL RK | 38 |
| Cecropin A (D. melanogaster) | GWLKKIGKKIERVGQHTRDATIQGLGIPQQAANV AATARG | 39 |
| Buforin II | TRSSRAGLQFPVGRVHRLLRK | 4 |
| Sarcotoxin IA | GWLKKIGKKIERVGQHTRDATIQGLGIAQQAANV AATAR | 40 |
| Apidaecin | ANRPVYIPPPRPPHPRL | 41 |
| Ascaphine 5 | GIKDWIKGAAKKLIKTVASHIANQ | 42 |
| Nigrocine 2 | GLLSKVLGVGKKVLCGVSGLVC | 43 |
| Pseudin 1 | GLNTLKKVFQGLHEAIKLINNHVQ | 44 |
| Ranalexin | FLGGLIVPAMICAVTKKC | 45 |
| Melittin | GIGAVLKVLTTGLPALISWIKRKRQQ | 46 |
| Lycotoxin 1 | IWLTALKFLGKHAAKKLAKQQLSKL | 47 |
| Parasin 1 | KGRGKQGGKVRAKAKTRSS | 48 |
| Buforin I | AGRGKQGGKVRAKAKTRSSRAGLQFPVGRVHRLL RKGNY | 49 |
| Dermaseptin 1 | ALWKTMLKKLGTMALHAGKAALGAAADTISQGTQ | 50 |
| Bactenecin 1 | RLCRIVVIRVCR | 51 |
| Thanatin | GSKKPVPIIYCNRRTGKCQRM | 52 |
| Brevinin 1T | VNPIILGVLPKVCLITKKC | 53 |
| Ranateurin 1 | SMLSVLKNLGKVGLGFVACKINIKQC | 54 |
| Esculentin 1 | GIFSKLGRKKIKNLLISGLKNVGKEVGMDVVRTG IKIAGCKIKGEC | 55 |
| Tachyplesin | RWCFRVCYRGICYRKCR | 56 |
| Androctonin | RSVCRQIKICRRRGGCYYKCTNRPY | 57 |
| alpha-defensin | DCYCRIPACIAGERRYGTCIYQGRLWAFCC | 58 |
| beta-defensin | NPVSCVRNKGICVPIRCPGSMKQIGTCVGRAVKC CRKK | 59 |
| theta-defensin | GFCRCLCRRGVCRCICTR | 60 |
| defensin (sapecin A) | ATCDLLSGTGINHSACAAHCLLRGNRGGYCNGKA VCVCRN | 61 |
| Thionin (crambin) | TTCCPSIVARSNFNVCRIPGTPEAICATYTGCII IPGATCPGDYAN | 62 |
| defensin from radish | QKLCQRPSGTWSGVCGNNNACKNQCIRLEKARHG SCNYVFPAHCICYFPC | 63 |
| Drosomycin | DCLSGRYKGPCAVWDNETCRRVCKEEGRSSGHCS PSLKCWCEGC | 64 |
| Hepcidin | DTHFPICIFCCGCCHRSKCGMCCKT | 65 |
| Bac 5 | RFRPPIRRPPIRPPFYPPFRPPIRPPIFPPIRPP FRPPLGRPFP | 66 |
| PR-39 | RRRPRPPYLPRPRPPPFFPPRLPPRIPPGFPPRF PPRFP | 67 |
| Pyrrhocoricin | VDKGSYLPRPTPPRPIYNRN | 68 |
| Histatin 5 | DSHAKRHHGYKRKFHEKHHSHRGY | 69 |
| ECP19 | RPPQFTRAQWFAIQHISLN | 70 |
| MSI-594 | GIGKFLKKAKKGIGAVLKVLTTG | 71 |
| TL-ColM | METLTVHAPSPSTNLPSYGNGAFSLSAPHVPGAG P | 72 |
| SBO | KLKKIAQKIKNFFAKLVA | 73 |
| Macedocin | GKNGVFKTISHECHLNTWAFLATCCS | 74 |
| Macedocin (Trunc) | GKNGVFKTISHECHLNTWAFLA | 75 |
| D16 | ACKLKSLLKTLSKAKKKKLKTLLKALSK | 76 |
| CPF-C1 | GFGSLLGKALRLGANVL | 77 |
| TL-ColM(-Met) | ETLTVHAPSPSTNLPSYGNGAFSLSAPHVPGAGP | 78 |
| TM-174E | LISKGWPYLLVVVLGATIYFWGNSNG | 79 |
| ECP45 | RPPQFTRAQWFAIQHISLNPPRCTIAMRAINNYR WRCKNQNTFLR | 80 |
| ColicinE3_1-51 (S37F) | SGGDGRGHNTGAHSTSGNINGGPTGLGVGGGASD GFGWSSENNPWGGGSG | 81 |
| ColicinE3_1-69 (S37F) | SGGDGRGHNTGAHSTSGNINGGPTGLGVGGGASD GFGWSSENNPWGGGSGSGIHWGGGSGHGNGGGNG | 82 |
| ColicinD_1-53 | SDYEGSGPTEGIDYGHSMVVWPSTGLISGGDVKP GGSSGIAPSMPPGWGDYS | 83 |
| | HPQYNQR | 100 |

The second amino acid sequence stretch may be a sushi peptide which is described by Ding JL, Li P, Ho B Cell Mol Life Sci. 2008 Apr;65(7-8):1202-19. The Sushi peptides: structural characterization and mode of action against Gram-negative bacteria. Especially preferred is the sushi 1 peptide according to SEQ ID NO: 84. Other preferred sushi peptides are sushi peptides S1 and S3 and multiples thereof (Tan et al, FASEB J. 2000 Sep;14(12):1801-13).

In particular in cases where the inventive polypeptide is to be recombinantly expressed by a host cell, it is preferred if the inventive polypeptide comprises a methionine residue at the N-terminus.

The inventive polypeptide may comprise additionally one or more tag sequences. Such tag sequence may for example be located at the N- or C-terminus of the inventive polypeptide. In a preferred embodiment, the one or more tag sequence is located on the C-terminal side of the polypeptide, e.g. directly at the C-terminus. The one or more tag sequences may be linked for example directly or via a short linker to the rest of the inventive polypeptide (see above). Numerous examples for tags are known in the art, some of which have already been mentioned above. In the context of the present invention a particularly preferred tag sequence is a His-tag, preferably a His tag according to SEQ ID NO: 91.

The length of the polypeptide according to present invention is in principle not limited, but preferably the length will not be excessively large. Preferably, a polypeptide according to the present invention has an overall length not exceeding about 400 amino acids, preferably not exceeding about 350 amino acids.

Polypeptides according to the present invention are polypeptides comprising the amino acid sequence of SEQ ID NO: 101, SEQ ID NO: 102, SEQ ID NO: 103 or SEQ ID NO: 105.

A polypeptide according to the present invention is preferably characterized by the ability to degrade the peptidoglycan of *Enterococcus* bacteria. If the enzyme is active, degradation of the peptidoglycan layer will lead to a drop of turbidity, which can be measured photometrically (see for example Briers et al., J. Biochem. Biophys Methods 70: 531-533, (2007).

The present invention does also relate to nucleic acids encoding one or more inventive polypeptides of the present invention. The inventive nucleic acid may take all forms conceivable for a nucleic acid. In particular the nucleic acids according to the present invention may be RNA, DNA or hybrids thereof. They may be single-stranded or doublestranded. The may have the size of small transcripts or of entire genomes, such as a bacteriophage genome. As used herein, a nucleic acid encoding one or more inventive polypeptides of the present invention may be a nucleic acid reflecting the sense strand. Likewise, the antisense strand is also encompassed. The nucleic acid may encompass a heterologous promotor for expression of the inventive polypeptide.

An inventive nucleic acid may comprise at least one sequence selected from the following group of sequences:
i) a nucleic acid sequence according to SEQ ID NO: 95;
ii) a nucleic acid sequence according to SEQ ID NO: 96;
iii) a nucleic acid sequence according to SEQ ID NO: 97; and
iv) a derivative of any one of SEQ ID NO: 95, SEQ ID NO: 96, or SEQ ID NO: 97 encoding the same amino acid sequence as SEQ ID NO: 95, SEQ ID NO: 96, or SEQ ID NO: 97, respectively.

In a further aspect, the present invention relates to a vector, which comprises a nucleic acid according to the present invention. Such vector may for example be an expression vector allowing for expression of an inventive polypeptide. Said expression may be constitutive or inducible. The vector may also be a cloning vector comprising the nucleic acid sequence of an inventive polypeptide for cloning purposes.

In a further aspect, the present invention relates to a host cell comprising a polypeptide according to the present invention, a nucleic acid according to the present invention, and/or a vector according to the present invention. The host cells may be selected in particular from the group consisting of bacterial cells and yeast cells. Particularly preferred host cells are *E. coli* cells.

In a further aspect, the present invention relates to composition comprising a polypeptide according to the present invention. Preferably, a composition according to the present invention comprises a pharmaceutical acceptable diluent, excipient or carrier. Such composition may be a pharmaceutical composition. Furthermore, a composition according to the present invention may be a bone cement comprising a polypeptide according to the present invention. The composition according to the present invention may also additionally encompass biomaterial, e.g. biomaterial as used in orthopedic applications. A person skilled in the art will be readily aware of a large number of possible materials in this respect.

In a further aspect the present invention relates to a polypeptide according to the present invention and/or a composition according the present invention for use in a method for treatment of the human or animal body by surgery or therapy or in diagnostic methods practiced on the human or animal body.

The present invention also relates to a polypeptide according to the present invention (and likewise a composition according to the present invention) for use in a method of treatment or prevention of infections caused by bacteria of the genus *Enterococcus.* In particular, the present invention relates to a polypeptide according to the present invention (and likewise a composition according to the present invention) for use in a method of treatment or prevention of infections caused by bacteria of the genus *Enterococcus,* wherein the polypeptide comprises a sequence selected from the following group of sequences:
i) the amino acid sequence according to SEQ ID NO: 101;
ii) the amino acid sequence according to SEQ ID NO: 102;
iii) the amino acid sequence according to SEQ ID NO: 103; and
iv) the amino acid sequence according to SEQ ID NO: 105.

Disclosure set out above for the inventive polypeptide is contemplated for the polypeptide (and the composition comprising such polypeptide) for use in a method of treatment or prevention of infections caused by bacteria of the genus *Enterococcus* as well.

Regarding the aspect of using a polypeptide according to the present invention and/or a composition according the present invention in a method for treatment of the human or animal body by surgery or therapy or in diagnostic methods practiced on the human or animal body (e.g. for the treatment and prevention of bacterial infections), the inventors specifically contemplate to use said polypeptide and composition at about physiological pH, e.g. at a pH of about 7.2 to 7.6, more preferably at a pH of about 7.4.

In a further aspect the present invention relates to the use of a polypeptide according to the present invention (and/or a composition according to the present invention) for disinfecting inanimate surfaces, compositions and/or objects, in particular in the nosocomial environment or in a doctor's office. Preferably, this is done at about physiological pH, e.g. at a pH of about 7.2 to 7.6, more preferably at a pH of about 7.4.

In a further aspect, the present invention relates to the use of a polypeptide according to the present invention (and/or a composition according to the present invention) for preventing contamination of inanimate surfaces, compositions and/or objects with bacteria, in particular for preventing contamination with *Enterococcus faecalis* and/or *Enterococcus faecium* bacteria. Again, this is preferably done at about physiological pH, e.g. at a pH of about 7.2 to 7.6, more preferably at a pH of about 7.4.

Disclosure set out above for the inventive polypeptide is contemplated for the uses according to the present invention as well.

In a further aspect, the present invention relates to a method for disinfecting inanimate surfaces, compositions and/or objects, in particular in the nosocomial environment or in a doctor's office, wherein the method comprises contacting the inanimate surfaces, compositions and/or objects with a polypeptide according to the present invention ( and/or a composition according to the present invention). This method is preferably carried out at about physiological pH, e.g. at a pH of about 7.2 to 7.6, more preferably at a pH of about 7.4.

In a further aspect, the present invention relates to a method for preventing contamination of inanimate surfaces, compositions and/or objects with bacteria, in particular for preventing contamination with *Enterococcus faecalis* and/or *Enterococcus faecium* bacteria, wherein the method comprises contacting the inanimate surfaces, compositions and/or objects with a polypeptide according to the present invention (and/or a composition according to the present invention). This method is also preferably carried out under pH conditions reflecting about a physiological pH, e.g. at a pH of about 7.2 to 7.6, more preferably at a pH of about 7.4.

Disclosure set out above for the inventive polypeptide is contemplated for the inventive method for disinfecting and the inventive method for preventing contamination as well.

### Examples

In the following, specific examples illustrating various embodiments and aspects of the invention are presented.

### Example 1: Increased antibacterial activity on E. faecalis bacteria compared to the wildtype endolysin

*E. faecalis* bacteria were grown over night in LB (Luria-Bertani) medium and diluted 1:10 in same medium. At optical density OD₆₀₀ of about 0.6 bacteria were pelleted, washed in buffer (10 mM HEPES, pH 7.4) and diluted 1:10 in same buffer. 50 µl bacteria solution was mixed with 50 µl of protein solution (10 µg in 20 mM HEPES, 300 mM NaCl, pH 7.4) or control (20 mM HEPES, 300 mM NaCl, pH 7.4). The proteins used were the wildtype endolysin (wt) according to SEQ ID NO:1 and a polypeptide comprising SEQ ID NO: 93 (not claimed). Samples were incubated for 60 min at 37°C with gently agitation. 25 µl of an undiluted sample and a 1:10 dilution series in 1 x PBS buffer was plated on LB agar plates which were incubated over night at 37°C. Colonies were counted and the growth reduction was determined.

Table 3 below shows the growth reduction of *E. faecalis* HC-1909-5 after incubation with the wildtype endolysin and the polypeptide comprising SEQ ID NO: 93 (not claimed). Incubation with endolysin led to a bacterial elimination of 99,99 % (4 log). More than 99,999 % (≥ 5 log) was achieved after incubation with the fusion protein. Four 1:10 dilutions were done leading to a test counting limit of 5 log reduction.

**Table 3:**

| Protein | |
|---|---|
| Wt endolysin | **4 log** |
| SEQ ID NO:93 | **≥ 5 log** |

### Example 2: Increased antibacterial activity on E. faecalis bacteria compared to the wildtype endolysin over broad pH range

*E. faecalis* bacteria were grown over night in LB (Luria-Bertani) medium and diluted 1:10 in same medium. At optical densitiy OD₆₀₀ of about 0.6 bacteria were pelleted, washed in different buffers. The buffers used were the following: 100 mM Malic acid disodium salt, pH 5 and pH 6 and mixtures of 1 M Na₂HPO₄ and 1 M NaH₂PO₄ for pH 7.4 and pH 8. Bacteria were then diluted 1:10 in different buffers (look above). 50 µl bacteria solution was mixed with 50 µl of protein solution (10 µg in 20 mM HEPES, 300 mM NaCl, pH 7.4) or control (20 mM HEPES, 300 mM NaCl, pH 7.4). The proteins used were the wildtype endolysin (wt) according to SEQ ID NO:1 and a polypeptide comprising SEQ ID NO: 93 (not claimed). The end pH values after mixing bacteria and proteins were the following: pH 5.25, pH 6.5, pH 7.4 and pH 7.75. Samples were incubated for 60 min at 37°C with gently agitation. 25 µl of an undiluted sample and a 1:10 dilution series in 1 x PBS buffer was plated on LB agar plates which were incubated over night at 37°C. Colonies were counted and the growth reduction was determined. Four 1:10 dilutions were done leading to a test counting limit of 5 log reduction.

Table 4 below shows the growth reduction of *E. faecalis* strain HC-1909-5 under different pH conditions after incubation with the wildtype endolysin and the polypeptide comprising SEQ ID NO: 93 (not claimed). 99-99.9 % (2-3 log) bacterial elimination were visible for the wildtype endolysin from pH 5.25 - pH 7.75 with decreasing activity at basic pH. 99.99-99.997 % (4-4.5 log) bacterial elimination was reached with the fusion protein with only slightly decreased activity at basic pH.

**Table 4:**

| [pH] | wildtype endolysin | SEQ ID NO: 93 (not claimed) | SEQ ID NO: 102 |
|---|---|---|---|
| 5,25 | **3 log** | **4.5 log** | **3.5 log** |
| 6 | **3 log** | **4.5 log** | **4 log** |
| 7,4 | **2 log** | **4.5 log** | **3.5 log** |
| 7,75 | **2.5 log** | **4 log** | **2.5 log** |

Similar results (e.g. essentially constant anti-bacterial activity over a broad pH range, in particular without significant activity loss at a physiological pH of 7.4) can also be achieved with other polypeptides, such as polypeptides comprising additional a His-tag (e.g. SEQ ID NO: 94 or SEQ ID NO: 103; not claimed), or with polypeptides comprising the cell wall binding domain (CBD) of SEQ ID NO:1 and an additional catalytic domain, e.g. a sequence according to SEQ ID NO:104. An example for such polypeptide is a polypeptide comprising SEQ ID NO: 105.

### Example 3: Broad antibacterial activity against diverse set of Enterococci strains

Bacteria were grown over night in LB (Luria-Bertani) medium and diluted 1:10 in same medium. At optical density OD₆₀₀ of about 0.6 bacteria were pelleted, washed in buffer (10 mM HEPES, pH 7.4) and diluted 1:10 in same buffer. 50 µl bacteria solution was mixed with 50 µl of protein solution (10 µg in 20 mM HEPES, 300 mM NaCl, pH 7.4) or control (20 mM HEPES, 300 mM NaCl, pH 7.4). The proteins used were the wildtype endolysin (wt) according to SEQ ID NO:1 and a polypeptide comprising SEQ ID NO: 93 (not claimed). Samples were incubated for 60 min at 37°C with gently agitation. 25 µl of an undiluted sample and a 1:10 dilution series in 1 x PBS buffer was plated on LB agar plates which were incubated over night at 37°C. Colonies were counted and the growth reduction was determined. Four 1:10 dilutions were done leading to a test counting limit of 5 log reduction.

Table 5 below shows the growth reduction of different *Enterococcus faecalis, Enterococcus faecium* and other *Enterococcus* strains. On average, 99.9987 % (4.9 log) bacterial elimination was determined.

**Table 5:**

| **Strain** | **Growth reduction** |
|---|---|
| *E. faecalis* HC-1909-5 | ≥ 5 log |
| *E. faecium* NCIMB 11181 | ≥ 5 log |
| *E. faecalis* DSM 20478 | ≥ 5 log |
| *E. faecalis* va96529 | ≥ 5 log |
| *E. faecium* va80443 | ≥ 5 log |
| *E. faecalis* 12470 | ≥ 5 log |
| *E. faecium* 13368 | ≥ 5 log |
| *E. faecalis* NWV 16205 | ≥ 5 log |
| *E. faecalis* NMV 10462 | ≥ 5 log |
| *E. faecium* 90-2 | ≥ 5 log |
| *E. faecalis* NWV 21315 | ≥ 5 log |
| *E. faecalis* NWV 16205 | ≥ 5 log |
| *E. faecium* NWV 6818 | ≥ 5 log |
| *E. faecium* NWV 8780 | ≥ 5 log |
| *E. faecalis* DSM 20478 | ≥ 5 log |
| *E. faecium* 13372 | ≥ 5 log |
| *E. faecium* 13371 | 5 log |
| *E. faecium* 13370 | ≥ 5 log |
| *E. faecium* 13369 | ≥ 5 log |
| *E. faecalis* 13350 | ≥ 5 log |
| *E. faecalis* 13293 | ≥ 5 log |
| *E. faecalis* 12643 | ≥ 5 log |
| *E. faecalis* 12583 | ≥ 5 log |
| *E. faecalis* ur10856 | ≥ 5 log |
| *E. faecalis* va32880_3 | 5 log |
| *E*. spec. Colja 9 | 4 log |
| *E. faecalis* DSM 12956 | ≥ 5 log |
| *E. faecalis* DSM 2981 | ≥ 5 log |
| *E. faecalis* DSM 2570 | ≥ 5 log |
| *E. faecalis* DSM 6134 | 4 log |
| *E. faecalis* Bobby 2 | ≥ 5 log |
| *E*. spec. 02.15-1.54 | 4 log |
| *E. faecalis* | ≥ 5 log |

## Claims

1. Polypeptide comprising:
i) the amino acid sequence of SEQ ID NO: 101;
ii) the amino acid sequence of SEQ ID NO: 102;
iii) the amino acid sequence of SEQ ID NO: 103; or
iv) the amino acid sequence of SEQ ID NO: 105.

2. The polypeptide according to claim 1, wherein the polypeptide degrades the peptidoglycan of Enterococcus bacteria, in particular of *Enterococcus faecalis* and/or *Enterococcus faecium* bacteria.

3. The polypeptide according to claim 1 or claim 2 wherein the polypeptide is at an about physiological pH, such as pH 7.4, more active than the wildtype enzyme (SEQ ID NO:1) and/or exhibits essentially the same or increased activity at about physiological pH compared to more acidic pH, such as pH 5.25 or 6.

4. A composition comprising a polypeptide of any one of the preceding claims and a pharmaceutically acceptable carrier, diluent or excipient.

5. The composition according to claim 4, wherein the composition is bone cement or comprises biomaterial.

6. A polypeptide according to any one of claims 1 to 3 or a composition according to any one of claims 4 to 5 for use in a method for treatment of the human or animal body by surgery or therapy or in diagnostic methods practiced on the human or animal body, in particular wherein the polypeptide or composition is used for the treatment or prevention of bacterial infections, in particular for the treatment or prevention of bacterial infections with *Enterococcus faecalis* and/or *Enterococcus faecium* bacteria.

7. The polypeptide or composition for use according to claim 6, wherein the polypeptide or composition is used at about physiological pH, e.g. at a pH of about 7.2 to 7.6, more preferably at a pH of about 7.4.

8. Use of a polypeptide according to any one of claims 1 to 3 or a composition according to claim 4 for disinfecting inanimate surfaces, compositions and/or objects, in particular in the nosocomial environment or in a doctor's office, or for preventing contamination of inanimate surfaces, compositions and/or objects with bacteria, in particular for preventing contamination with *Enterococcus faecalis* and/or *Enterococcus faecium* bacteria, in particular wherein the polypeptide or composition is used at about physiological pH, e.g. at a pH of about 7.2 to 7.6, more preferably at a pH of about 7.4.

9. Nucleic acid encoding a polypeptide according to any one of claims 1 to 3.

10. Vector comprising a nucleic acid according to claim 9.

11. Host cell comprising a polypeptide according to any one of claims 1 to 3, a nucleic acid according to claim 9, and/or a vector according to claim 10.

## Patentansprüche

1. Polypeptid umfassend:
i) die Aminosäuresequenz der SEQ ID NO: 101;
ii) die Aminosäuresequenz der SEQ ID NO: 102;
iii) die Aminosäuresequenz der SEQ ID NO: 103; oder
iv) die Aminosäuresequenz der SEQ ID NO: 105.

2. Das Polypeptid nach Anspruch 1, wobei das Polypeptid das Peptidoglykan von Enterococcus-Bakterien, insbesondere von *Enterococcus faecalis-* und/oder *Enterococcus faecium-*Bakterien*,* abbaut.

3. Das Polypeptid nach Anspruch 1 oder Anspruch 2, wobei das Polypeptid bei einem in etwa physiologischen pH, wie pH 7,4, aktiver als das Wildtypenzym (SEQ ID NO:1) ist und/oder bei in etwa physiologischem pH im Wesentlichen die gleiche oder eine erhöhte Aktivität im Vergleich zu saurerem pH, wie pH 5,25 oder 6, aufweist.

4. Eine Zusammensetzung umfassend ein Polypeptid nach einem der vorhergehenden Ansprüche und einen pharmazeutisch verträglichen Träger, ein pharmazeutisch verträgliches Verdünnungsmittel oder einen pharmazeutisch verträglichen Hilfsstoff.

5. Die Zusammensetzung nach Anspruch 4, wobei die Zusammensetzung Knochenzement ist oder Biomaterial umfasst.

6. Ein Polypeptid nach einem der Ansprüche 1 bis 3 oder eine Zusammensetzung nach einem der Ansprüche 4 bis 5 zur Verwendung in einem Verfahren zur chirurgischen oder therapeutischen Behandlung des menschlichen oder tierischen Körpers oder in einem Diagnostizierverfahren, das am menschlichen oder tierischen Körper vorgenommen wird, insbesondere wobei das Polypeptid oder die Zusammensetzung zur Behandlung oder Prävention bakterieller Infektionen, insbesondere zur Behandlung oder Prävention von bakteriellen Infektionen mit *Enterococcus faecalis-* und/oder *Enterococcus faecium-*Bakterien*,* verwendet wird.

7. Das Polypeptid oder die Zusammensetzung zur Verwendung nach Anspruch 6, wobei das Polypeptid oder die Zusammensetzung bei etwa physiologischem pH, z.B. bei einem pH von etwa 7,2 bis 7,6, noch bevorzugter bei einem pH von etwa 7,4, verwendet wird.

8. Verwendung eines Polypeptids nach einem der Ansprüche 1 bis 3 oder einer Zusammensetzung nach Anspruch 4 zur Desinfektion unbelebter Oberflächen, Zusammensetzungen und/oder Objekte, insbesondere im nosokomialen Umfeld oder in einer Arztpraxis, oder zur Verhinderung der Kontamination unbelebter Oberflächen, Zusammensetzungen und/oder Objekte mit Bakterien, insbesondere zur Verhinderung der Kontamination mit *Enterococcus faecalis-* und/oder *Enterococcus faecium-*Bakterien, insbesondere wobei das Polypeptid oder die Zusammensetzung bei etwa physiologischem pH, z.B. bei einem pH von etwa 7,2 bis 7,6, noch bevorzugter bei einem pH von etwa 7,4, verwendet wird.

9. Nukleinsäure, die für ein Polypeptid nach einem der Ansprüche 1 bis 3 kodiert.

10. Vektor umfassend eine Nukleinsäure nach Anspruch 9.

11. Wirtszelle umfassend ein Polypeptid nach einem der Ansprüche 1 bis 3, eine Nukleinsäure nach Anspruch 9, und/oder einen Vektor nach Anspruch 10.

## Revendications

1. Polypeptide comprenant :
i) la séquence d'acides aminés de la SEQ ID NO : 101 ;
ii) la séquence d'acides aminés de la SEQ ID NO : 102 ;
iii) la séquence d'acides aminés de la SEQ ID NO : 103 ; ou
iv) la séquence d'acides aminés de la SEQ ID NO : 105.

2. Polypeptide selon la revendication 1, lequel polypeptide dégrade le peptidoglycane de bactéries Enterococcus, en particulier de bactéries *Enterococcus faecalis* et/ou *Enterococcus faecium.*

3. Polypeptide selon la revendication 1 ou la revendication 2, lequel polypeptide est, aux environs du pH physiologique, tel que pH 7,4, plus actif que l'enzyme de type sauvage (SEQ ID NO : 1) et/ou présente une activité pratiquement identique ou accrue aux environs du pH physiologique en comparaison avec un pH plus acide, tel que pH 5,25 ou 6.

4. Composition comprenant un polypeptide de l'une quelconque des revendications précédentes et un véhicule, diluant ou excipient pharmaceutiquement acceptable.

5. Composition selon la revendication 4, laquelle composition est un cément osseux ou comprend un biomatériau.

6. Polypeptide selon l'une quelconque des revendications 1 à 3 ou composition selon l'une quelconque des revendications 4 et 5 pour une utilisation dans une méthode de traitement du corps humain ou animal par chirurgie ou thérapie ou dans des méthodes de diagnostic pratiquées sur le corps humain ou animal, en particulier lequel polypeptide ou laquelle composition est utilisé pour le traitement ou la prévention d'infections bactériennes, en particulier pour le traitement ou la prévention d'infections bactériennes par des bactéries *Enterococcus faecalis* et/ou *Enterococcus faecium.*

7. Polypeptide ou composition pour une utilisation selon la revendication 6, lequel polypeptide ou laquelle composition est utilisé aux alentours du pH physiologique, par exemple à un pH d'environ 7,2 à 7,6, mieux encore à un pH d'environ 7,4.

8. Utilisation d'un polypeptide selon l'une quelconque des revendications 1 à 3 ou d'une composition selon la revendication 4 pour désinfecter des surfaces, compositions et/ou objets inanimés, en particulier dans l'environnement nosocomial ou au cabinet d'un médecin, ou pour empêcher une contamination de surfaces, compositions et/ou objets inanimés par des bactéries, en particulier pour empêcher une contamination par des bactéries *Enterococcus faecalis* et/ou *Enterococcus faecium,* en particulier dans laquelle le polypeptide ou la composition est utilisé aux alentours du pH physiologique, par exemple à un pH d'environ 7,2 à 7,6, mieux encore à un pH d'environ 7,4.

9. Acide nucléique codant un polypeptide selon l'une quelconque des revendications 1 à 3.

10. Vecteur comprenant un acide nucléique selon la revendication 9.

11. Cellule hôte comprenant un polypeptide selon l'une quelconque des revendications 1 à 3, un acide nucléique selon la revendication 9, et/ou un vecteur selon la revendication 10.
